# EUROPEAN PATENT SPECIFICATION

(11) **EP 0 328 355 B1**
(45) Date of publication and mention of the grant of the patent: **12.01.1994**
(21) Application number: 89301182.5
(22) Date of filing: 08.02.1989
(51) Int. Cl.: A61K 9/10

(54) **Emulsion compositions including amphipathic emulsifying agents**
Amphipatische Emulgiermittel enthaltende Emulsionzusammensetzungen
Compositions d'émulsions contenant des agents émulsionants amphipathiques

(30) Priority: 10.02.1988 US 154644
(43) Date of publication of application: 16.08.1989
(73) Proprietor: RICHARDSON VICKS, INC., Wilton Connecticut 06897 (US)
(72) Inventor: Puchalski, Eugene, Jersey City, NJ 07305 (US); Deckner, George, Westfield, NJ 07090 (US); Schneider, Emil F., Long Branch, NJ 07740 (US)
(74) Representative: Beresford, Keith Denis Lewis

(56) References cited:
- EP-A- 0 085 334
- EP-A- 0 215 313
- EP-A- 0 268 164
- FR-A- 1 352 236
- US-A- 1 033 299
- SOAP/COSMETICS/CHEMICAL SPECIALTIES, vol. 63, no. 5, May 1987, pp. 28, 29, 32, 33, 84, 85, New York, USA; R.Y. Lochhead et al.:"Hydrophobically Modified 'Carbopol' Resins"

## Description

This invention relates in general to the preparation of a variety of emulsion compositions, and more particularly, to emulsion compositions and methods for preparing these emulsion compositions which employ amphipathic emulsifying agents and high shear polymeric emulsion techniques. The emulsion compositions of this invention although applicable to a variety of applications, and particularly applicable to and are therefore described in connection with the preparation of such emulsion compositions useful for the prevention of diaper dermatitis and the like.

Diaper dermatitis is believed to be caused by the prolonged contact of the skin with body waste. The exact component or components of urine and feces responsible for diaper dermatitis has not been identified. Factors which have been suspected of causing diaper dermatitis include ammonia, moisture, bacteria, urine pH, and Candida albicans. Because these various suspected factors have different properties and require different therapies, the most effective method of treating diaper dermatitis has been the application of a topical protective barrier agent between the skin and the body waste.

Common protective topically applied barrier agents and the ingredients they contain include the following: Caldesene® Powder (calcium undecylenate, 10%, fragrance, and talc) and Ointment (petrolatum, 53.9%, and zinc oxide, 15%); Desitin® Ointment (zinc oxide, 40%, and cod liver oil, vitamins A and D, in a petrolatum-lanolin base, Leeming Division of Pfizer, Inc.); Balmex® Baby Powder (Balsan®, a specially purified balsam, Peru, zinc oxide, talc, starch, and calcium carbonate), Ointment (Balsan ®, vitamins A and D, zinc oxide and bismuth subnitrate in an ointment base containing silicone) and Lotion (Balsan®, lanolin oil, a nonsensitizing, dewaxed moisturizing fraction and silicone).

Other lotions, creams and ointments used in the treatment of diaper dermatitis and the active ingredients they contain include the following; Baby Magic Baby Lotion (Mennan) benzalkonium chloride; Johnson's Baby Cream (Johnson & Johnson) 2% dimethicone and Lotion; Diaparene Peri-Anal Medicated Ointment (Glenbrook Labs) 0.1% methylbenzethonium chloride; Suave Baby Care Skin Lotion (Helene Curtis); and Methakote DiaperRash Cream (Syntex) protein hydrolysate composed of L-leucine, isoleucine, L-methionine, L-phenylalanine, L-tyrosine, DL-methionine, cysteine hydrochloride, benzethonium chloride and talc, United States patent No. 3,061,512.

In addition to protective barrier agents, baby wipes are also used in the treatment of diaper dermatitis. Baby wipes are premoistened, disposable towelettes used primarily during diaper changes for cleansing. The wipe is usually constructed of non-woven wood pulp (approximately 85%) and another fiber such as polyester bonded with a styrene butadiene rubber latex. Wipes are generally moistened with water (over 95%) and contain various combinations of humectants, emollients, surfactants, preservatives and scents.

Baby wipes currently available and ingredients they contain include the following: Baby Fresh (Scott); Johnson's Baby Wash Cloths (Johnson & Johnson); Wet Ones (Lehn and Fink Products Group, Division of Sterling Drug, Inc.) natural aloe, United States patent Nos. 4,017,002 and 4,337,876; Diaperene Baby Wash Cloths with Lanolin (Glenbrook Labs, Division of Sterling Drug) United States patent No. 4,017,002; Tender Wipes (Young's Drug Products) benzethonium chloride; and Chubs Thick Baby Wipes (Lehn and Fink Products Group, Division of Sterling Drug, Inc.) aloe, United States patent Nos. 4,017,002 and 4,337,876;
Various combinations of the following ingredients are used in baby wipe products: water, SD alcohol 40, benzyl alcohol, propylene glycol, aloe vera gel, PEG-60 lanolin, PEG-75 lanolin, PEG-85 lanolin, sodium nonoxynol-9 phosphate, sorbic acid, oleth-lO, oleth-20, fragrance, citric acid, disodium phosphate, DH DM hydantoin, sodium phosphate, benzalkonium chloride, methylparaben, propylparaben, butylparaben, sodium hydroxide, octoxynol-9, simethicone, polysorbate-20, cocoamphocarboxyglycinate, methylchloroisothiazolinone, methylisothiazolinone, trisodium EDTA, Quaternium-15, lauramine oxide, mineral oil, glycerine, PEG-8 stearate, petrolatum, Quaternium-27 and 2-bromo-2-nitropropane-1,3-diol.

Treatment of diaper dermatitis usually requires the combination of wipes and a protective barrier agent. The wipes are used initially, for cleansing, and then a barrier agent is applied for skin protection. Baby wipes currently available do not leave after application substantive or wash-resistant residues to protect the skin. Barrier agents are generally greasy and unappealing and are not effective cleansing agents. In addition, the use of some barrier agents may actually promote excessive skin hydration which may result in increased skin friction thus aggravating diaper dermatitis.

TAKE OFF (Johnson & Johnson) is a wipe product which contains an emulsion composition formulated as a makeup remover but has no substantive or skin protective properties.

There is thus a need in the treatment of diaper dermatitis for a hybrid product which would effectively cleanse and also leave a substantive highly protective residue on the skin. It would also be desirable to be able to apply the hybrid product as a wipe. The product should cleanse and leave a protective residue which does not significantly reduce transepidermal moisture loss.

EP-A-85 334 describes an oil in water composition comprising 40-95% water and 0.01-5% of an emulsifier. However, it is concerned with the formation of dermatologically acceptable highly substantive topical oil in water emulsions which are particularly useful as vehicles for sunscreen compositions. The document discloses that substantivity can be achieved by incorporating a polyanhydride resin derived from a octadecene-1 and maleic anhydride. The document is not concerned with forming a stable concentrated or dilute oil-in-water emulsion of the kind described below.

EP-A-268 164 is concerned with oil-in-water emulsions which contain a modified polymer which is a co-polymer of a preponderant amount of an acrylic acid and a smaller amount of a long chain acrylate monomer. The emulsifiers are stated to be stable over a period of over one year at room temperature but exhibit quick breaking properties when in contact with an electrolyte or the skin, instantaneously coalescing and releasing the oil. The use of highly crosslinked polyacrylic acids in the formation of emulsions is disclosed but is said to be undesirable because the required quick breaking properties are not achieved.

"Hydrophobically Modified Carbopol Resins" by Lochhead, RY et al, in Soap Cosmetics Chemical Specialities, no. 5, May 1987, USA discloses a high viscosity oil-in-water emulsion based on high molecular weight crosslinked polymers of acrylic acid and not an oil-in-water emulsion which is dilutable with water using low shear polymeric emulsion forces to form a stable oil-in-water emulsion.

One aspect of the present invention provides a concentrated oil in water emulsion composition in which the oil is present in the range of from 3.0% to 40% by weight of said composition and is all silicone based, in which an amphipathic emulsifying agent is present in the range of from 0.02% to 2.0% by weight of the composition, and in which the emulsion has been formed under high shearing forces, the emulsion having in combination the properties that:
(a) it is stable when in concentrated form; and
(b) on dilution with water under low shearing forces it can form a stable diluted oil in water emulsion in which water is present in an amount of 70 to 99% by weight of said composition and which has a viscosity of less than 100 cps (1.0g/cm sec).

Preferably, the emulsifying agent is selected from the group consisting of carboxy vinyl polymers, octadecene-maleic anhydride copolymers, stearyl ether-maleic anhydride copolymers, alkyl stearate-valeric anhydride copolymers and C₁₂ - C₂₂ alkyl-substituted acrylic acid copolymers where the alkyl group is lauric, myristic, palmitic, stearic, oleic, linolenic or isostearic, or combinations thereof.

Advantageously, the emulsifying agent is present in the range of from 0.8% to 1.4% by weight of the composition.

Preferably, the oil is present in the range of 27% to 33% by weight of the composition.

Advantageously, the oil is selected from the group consisting of dimethicone, dimethicone and trimethylsiloxy silicate, phenyl dimethicone, stearoxy dimethicone, cyclomethicone, cyclomethicone and dimethicone, or combinations thereof.

Another aspect of the invention provides a method for making a concentrated oil in water emulsion having any of the features described above, which comprises:
(i) applying high shearing forces to oil in water emulsion forming materials of which 3.0 to 40% of the materials are oil, all of which oil is silicone based, and of which 0.02% to 2.0% by weight of the materials is an amphipathic emsulsifying agent; and
(ii) recovering a concentrated oil in water emulsion having the properties (a) and (b) defined above.

A further aspect of the invention provides a method for making a diluted oil in water emulsion, which comprises:
(a) diluting with water under low shearing forces a composition of the type described above or made by the method described above until water is present in an amount of 70 to 99% by weight of the composition; and
(b) recovering a diluted oil in water emulsion which is stable and which has a viscosity of less than 100 cps (1.0g/cm sec).

In another aspect the invention relates to the use of an emulsion composition of the type as described above in the manufacture of a lotion, cream, spray, gel or pre-moistened towelette ("baby-wipe") for the prophylaxis or treatment of diaper dermatitis.

The invention provides a method for the preparation of a great variety of emulsion compositions, in particular, for the preparation of compositions that cleanse and also leave a highly protective substantive residue on the skin. Such compositions are useful in a great variety of therapies, for example, in the treatment of diaper dermatitis.

The oil-in-water emulsion compositions are highly substantive, safe, stable and economical products.

The emulsifying agents which may be used in the present invention exhibit amphipathic properties and are capable of primary emulsification of oil-in-water emulsions. The emulsifiers should be capable of rapidly inverting or de-emulsifying the emulsion to form an oil film upon application to the skin.

For a better understanding of the invention, embodiments of it will now be further described by way of example.

In the following description "Carbopol", "Kathon" and "Silicone 200" are registered trade marks.

The emulsifying agents which are useful in the present invention include Carbopol (Carbomer) 940, Carbopol 934, Carbopol 941, Carbopol 1342 and Gulf Polymer P18 (octadecene/maleic anhydride copolymer) and combinations thereof. Carbopol represents a group of water soluable vinyl polymers such as carboxyvinyl polymers.

Other useful emulsifying agents include C₁₂-C₂₂ alkyl-substituted acrylic acid copolymers, either alone or in combination with the above emulsifying agents, where the alkyl group is lauric, myristic, palmitic, stearic, oleic, linolenic or isostearic. Still other useful emulsifying agents, either alone or in combination with the above emulsifying agents, include stearyl ether/maleic anhydride copolymers (Gantroz AN-8194) and allyl stearate/valeric anhydride copolymer (Mexomere PG).

The emulsifying agents which are useful in the present invention may be present in the range of from 0.02% to 2.0%, and preferably from 0.8% to 1.4%, by weight of the emulsion composition.

The oils (emollients or barrier aids) which are useful in the present invention include dimethicone (Dow Corning 200 Fluids), dimethicone and trimethylsiloxy silicate (Dow Corning 593 Fluid), phenyl dimethicone (Dow Corning 556 Fluid), stearoxy dimethicone (SWS 755 Wax), cyclomethicone (Dow Corning 344 or 345 Fluid, Union Carbide 7158 Fluid, GE SF1173, 1202 or 1204, Ganex U216 or U220), cyclomethicone and dimethicone (Dow Corning X2-1401) and dimethicone (GE SE30, 76 gums, ultra high molecular weight dimethicone, greater than 10¹² m²/s (1,000,000 centistokes (cs))) or combinations thereof. Dimethicone is a silicone oil of methylsiloxane polymers.

The oils which are useful in the present invention may be present in the range of from 3.0 to 40%, and preferably from 27% to 33%, by weight of the emulsion composition.

The emulsion compositions of the present invention are subjected to high shear polymeric emulsion forces. A turbine mixer and in-line homogenizer utilizing tandem rotor-stators (two turbines and mating stators in tandem on a single shaft) may be used for in-line continuous high-speed high-shear homogenizing-mixing, emulsifying and rapid dispersing, available from Greerco Corp. of Hudson, New Hampshire. Shaft-rotor speeds in the range of 5,000 to 15,000 may be used. A shaft-rotor speed of about 10,000 rpm is preferred.

Optional ingredients which may be used in the present invention include preservatives, antifungal agents, skin protectants, moistening/humectant agents, pH adjusters, powders and the like.

The preservatives which may be used in the present invention include Quaternium 15 (Dowicil 200), methyl paraben (Tegosept M), propyl paraben (Tegosept P), DHDM hydantoin (Glydant), benzyl alcohol, methylchloroisothiazolinone and methylisothiazolinone (Kathon CG), butyl paraben (Tegosept B), imidazolidinyl urea (Germall 115), diazolidinyl (Germall II), and sequestrene No. 2, No. 3 or No. 4 (Disodium EDTA, trisodium EDTA, tetrasodium EDTA, respectively). Sequestreone is a series of complexing agents and metal complexes general of ethylenediaminetetraacetic acid and salts.

The antifungal agents which may be used in the present invention include chlorobenzyl alcohol (Myacide SP), mycostatin (Nystatin), miconazole (Ohasept Extra), parachloro meta zylenol, undecyclenic acid, calcium undecyclenate and zinc undecyclenate.

The skin protectants which may be used in the present invention include allantoin, zinc carbonate, zinc oxide, zinc acetate, cocoa butter, shark liver oil, kaolin, calamine, aluminum hydroxide gel and glycerin.

The moistening/humectant agents which may be used in the present invention include glycerin, propylene glycol, PEG 8 (Carbowax 400), sorbitol (Sorbo 70%), polyglyceryl methacrylate and propylene glycol (Lubrajel), proline and sodium PCA (Agidew NSO).

The agents which may be used to adjust pH in the present invention include triethanolamine (TEA, Quadrox), sodium hydroxide, potassium hydroxide, citric acid, lactic acid, glucamine (Desamine) and arginine.

The powders which may be used in the present invention include cornstarch, rice starch, talc, mica, starch modified polyacrylic acid, nylon powder, silicone treated nylon and silicon treated talc.

A preferred emulsion composition comprises Silicone 200 (3^{.}5 x 10⁸ m²/s and 10⁹ m²/s (350 cs and 1,000 cs)) (5%), Carbopol 1342 (0.15%), Kathon CG (0.10%), 99% Triethanolamine (0.20%), and Sequestrene No. 2 (0.10%) in water (about 95%).

The emulsion compositions of the present invention may be applied with a wipe by impregnating or otherwise contacting the emulsion composition with a web which may be a cellulosic wipe.

Oil in water emulsion compositions prepared with amphipathic emulsifiers and subjected to high shear polymeric emulsification have advantages over emulsion compositions prepared using conventional techniques which include the following:
1. Emulsion compositions of the present invention de-emulsify when applied to the skin helping to increase the substantivity, or wash resistance, of the composition on the skin. This resulting protective substantive barrier significantly reduces wet and dry skin friction and protects the skin against the irritating effects of chemicals. Deemulsification of the compositions also results in a pleasant feel on the skin.
2. Emulsion compositions of the present invention may be prepared in a highly concentrated form resulting in significant cost advantage. The emulsion compositions may be prepared as a 10-fold concentrate and then diluted with water using low shear processing equipment to complete preparation of the composition. Both the emulsion concentrate and the diluted formula were found to be stable at 48°C, 25°C, 4°C and -15°C for over one month. Such emulsion stability is extremely difficult to achieve with compositions prepared by conventional emulsion technology, especially for lotions with viscosity less than 100 mPas (100 cps).
3. Emulsion compositions of the present invention require only very low concentrations of emulsifying agents (Carbopol 1342, 0.15%) to achieve emulsion stability. In addition to being economical, low emulsifying agent levels help reduce the irritation potential of the compositions. The primary skin irritation on 105 subjects was shown to be extremely low using the Repeat Insult Patch Test (RIPT). A chamber scarification test was also performed and the results showed that the emulsion compositions of the present invention were as gentle as physiological saline on human abraded skin.
4. Emulsion compositions of the present invention have significantly more uniform moisture profiles or gradients when absorbed onto a web than emulsion compositions prepared by conventional techniques.
5. Emulsion compositions of the present invention significantly improve the cosmetic properties of the web. The tactile properties on skin of the instant compositions impregnated on a wipe were judged to be greatly improved over that of other emulsion compositions impregnated on a wipe (soft, smooth, tacky, moisturised). Emulsion compositions of the present invention also showed a 34% reduction in the coefficient of friction on webs versus conventional emulsion compositions. This data correlated well with sensory panel testing data.
6. Emulsion compositions of the present invention do not significantly affect the physical strength of the web. Emulsions prepared by a conventional means may chemically interact with the web binder and cause a significant reduction in the physical strength of the web.

The methods employed to prepare the emulsion compositions of the present invention are applicable to the preparation of a great variety of emulsion compositions. Such compositions include low irritation, wash resistant lotions, creams, sprays and clear gels; high performance, unique feeling, skin treatment and sunscreen products; alcohol-free, solubilizer-free perfumes, colognes, after-shave products; ultra gentle baby products; hair conditioners; substantive toners; highly effective, gentle makeup removers; and many non-cosmetic products.

The following examples are illustrative:

### EXAMPLE 1

A particularly preferred emulsion composition in the form of a barrier lotion useful for the treatment of diaper dermatitis was prepared in accordance with the present invention having the following composition:

| 10- Fold Concentrate Formula | | |
|---|---|---|
| Trade Name | CTFA Name | Parts by Weight |
| Deionized Water | Water | 63.275 |
| 200 Fluid (3^{.}5 x 10⁸ m²/s (350 cs)) | Dimethicone | 24.000 |
| 200 Fluid (10⁹ m²/s (1000 cs)) | Dimethicone | 10.000 |
| Carbopol 1342 | Carbomer 1342 | 1.500 |
| Triethanolamine | TEA | .125 |
| Kathon CG | Methychloroisothiazolinone (and Methyisothiazolinone | .100 |

| Dilution Formula | | |
|---|---|---|
| Trade Name | CTFA Name | Parts by Weight |
| Deionized Water | Water | 89.60 |
| Concentrate | (See Above) | 10.00 |
| Disodium ethylene diamine tetraacetic acid | Disodium EDTA | .10 |
| Triethanolamine | TEA | .10 |
| Kathon CG | Methychloroisothiazolinone (and Methyisothiazolinone | . |

### Example 2

A preferred emulsion composition in the form of a barrier lotion useful for the treatment of diaper dermatitis was prepared in accord with the present invention having the following composition:

| Trade Name | CTFA Name | Per Cent | Range |
|---|---|---|---|
| Deionized Water | Water | 94.03 | 70-99 |
| Sequestrene No. 2 | Disodium EDTA | .10 | .01-.50 |
| Carbopol 1342 | Carbomer 1342 | .15 | .02-1.5 |
| Silicone 200 (3^{.}5 x 10⁸ m²/s (350 cs)) | Dimethicone | 2.40 | 1-10 |
| Silicone 200 (10⁹ m²/s (1000 cs)) | Dimethicone | 1.00 | .5-10 |
| Deionized Water | Water | 1.00 | |
| Kathon CG | Methychloroisothiazolinone (and Methyisothiazolinone | .11 | .02-.12 |
| Deionized Water | Water | 1.00 | |
| 99% Triethanolamine | TEA | .21 | .05-3.0 |

### Example 3

A preferred emulsion composition in the form of a barrier lotion concentrate useful for the treatment of diaper dermatitis was prepared in accord with the present invention having the following composition;

| Number | Trade Name | Percent |
|---|---|---|
| A-1 | Deionized Water | 65.768 |
| A-2 | Sequestrene NA₂ | 0.240 |
| B-3 | Deionized Water | 1.000 |
| B-4 | Triethanolamine 99% | 0.092 |
| C-5 | Kathon CG | 0.100 |
| D-6 | Volatile Silicone | 12.000 |
| D-7 | Carbopol 1342 | 1.100 |
| D-8 | Silicone 200 (3^{.}5 x 10⁸ | 12.000 |
| | m²/s (350 cs)) | |
| D-9 | Silicone 200 (10⁹ m²/s (1000 cs)) | 7.700 |

### MIXTURE D

Volatile Silicone (D-6) was charged to a feed kettle equipped with a propeller mixer. Vigorous mixing was commenced. Carbopol 1342 (D-7) was added and when completely wet-out, silicone 200 (3^{.}5 x 10⁸ m²/s (350 cs)) (D-8) was added and propellar mixed 5 minutes. Silicone 200 (10⁹ m²/s (1000 cs)) (D-9) was then added to the mixture and propellar mixed.

### MIXTURE A

Deionized water was charged to the main kettle equipped with a turbine mixer and in-line homogenizer utilizing tandem rotor-stators. Turbine mixing was commenced ( 10,000 rpm), Sequestrene NA₂ was added (A-2), and turbine mixing was continued 5 minutes.

### MIXTURE B

Deionized water (B-3) and triethanolamine (B-4) were premixed with the propellar mixer. When mixture A and mixture B were uniform, mixture B was added to mixture A and turbine mixed 10-15 minutes. When the mixture of A, B and mixture D were uniform, Kathon CG (C-5) was added to the mixture of A and B. The mixture of A and B and C was turbine mixed 1 minute. The recirculation of mixture A and B and C thru the in-line homogenizer was commenced. Mixture D was introduced through an "I" connection on the recirculation loop. When the addition was complete, the feed kettle was rinsed with the withheld silicone. Turbine mixing and recirculation was continued thru the in-line homogenizer for 60 minutes or until the Carbopol was dispersed. The temperature was kept below 30°C. When the mixture was uniform, the recirculation thru the in-line homogenizer was stopped. Turbine mixing was continued 60 minutes. The mixture was pumped and strained.

## Claims

1. A concentrated oil in water emulsion composition in which the oil is present in the range of from 3.0% to 40% by weight of said composition and is all silicone based, in which an amphipathic emulsifying agent is present in the range of from 0.02% to 2.0% by weight of the composition, and in which the emulsion has been formed under high shearing forces, the emulsion having in combination the properties that:
(a) it is stable when in concentrated form; and
(b) on dilution with water under low shearing forces it can form a stable diluted oil in water emulsion in which water is present in an amount of 70 to 99% by weight of said composition and which has a viscosity of less than 100 cps (1.0g/cm sec).

2. An emulsion according to claim 1, wherein the emulsifying agent is selected from the group consisting of carboxy vinyl polymers, octadecene-maleic anhydride copolymers, stearyl ether-maleic anhydride copolymers, alkyl stearate-valeric anhydride copolymers and C₁₂- C₂₂ alkyl-substituted acrylic acid copolymers where the alkyl group is lauric, myristic, palmitic, stearic, oleic, linolenic or isostearic, or combinations thereof.

3. An emulsion according to claim 1 or 2, wherein the emulsifying agent is present in the range of from 0.8% to 1.4% by weight of the composition.

4. An emulsion according to claim 1, 2 or 3, wherein the oil is present in the range of 27% to 33% by weight of the composition.

5. An emulsion according to any one of the preceding claims, wherein the oil is selected from the group consisting of dimethicone, dimethicone and trimethylsiloxy silicate, phenyl dimethicone, stearoxy dimethicone, cyclomethicone, cyclomethicone and dimethicone, or combinations thereof.

6. A method for making a concentrated oil in water emulsion having the features of any of claims 1 to 5, which comprises:
(a) applying high shearing forces to oil in water emulsion forming materials of which 3.0 to 40% of the materials are oil, all of which oil is silicone based, and of which 0.02% to 2.0% by weight of the materials is an amphipathic emsulsifying agent; and
(b) recovering a concentrated oil in water emulsion having the properties (a) and (b) defined in claim 1.

7. A method for making a diluted oil in water emulsion, which comprises:
(a) diluting with water under low shearing forces a composition according to any of claims 1 to 5 or made by the method of claim 6 until water is present in an amount of 70 to 99% by weight of the composition; and
(b) recovering a diluted oil in water emulsion which is stable and which has a viscosity of less than 100 cps (1.0g/cm sec).

8. Use of an emulsion composition as claimed in any of claims 1 to 5 or made by the method of claim 6 or 7 in the manufacture of a lotion, cream, spray, gel or pre-moistened towelette ("baby-wipe") for the prophylaxis or treatment of diaper dermatitis.

## Patentansprüche

1. Konzentrierte Öl-in-Wasser-Emulsionszusammensetzung, in der das Öl in einem Anteil von 3,0 bis 40 Gew.-% der Zusammensetzung vorliegt und vollständig auf Siliconbasis beruht, in der ein amphipatisches Emulgiermittel in einem Anteil von 0,02 bis 2,0 Gew.-% der Zusammensetzung vorhanden ist und in der die Emulsion unter Einwirkung hoher Scherkräfte gebildet worden ist, wobei die Emulsion in Kombination die folgenden Eigenschaften aufweist:
a) sie ist in konzentrierter Form stabil; und
b) bei Verdünnung mit Wasser unter geringen Scherkräften kann sie eine stabile verdünnte Öl-in-Wasser-Emulsion bilden, in der das Wasser in einem Anteil von 70 - 99 Gew.-% der Zusammensetzung vorliegt und die eine Viskosität von weniger als 100 Cp (1,0 g/cm x sec) aufweist.

2. Emulsion nach Anspruch 1, wobei das Emulgiermittel aus der Gruppe Carboxyvinyl-Polymere, Octadecen-Maleinsäureanhydrid-Copolymere, Stearylether-Maleinsäureanhydrid-Copolymere, Alkylstearat-Valeriansäureanhydrid-Copolymere und C₁₂-C₂₂-alkyl-substituierte Acrylsäure-Copolymere, wobei es sich bei der Alkylgruppe um Lauryl, Myristyl, Palmityl, Stearyl, Oleyl, Linoleyl oder Isostearyl oder um Kombinationen davon handelt, ausgewählt ist.

3. Emulsion nach Anspruch 1 oder 2, wobei das Emulgiermittel in einem Anteil von 0,8 bis 1,4 Gew.-% der Zusammensetzung enthalten ist.

4. Emulsion nach Anspruch 1, 2 oder 3, wobei das Öl in einem Anteil von 27 - 33 Gew.-% der Zusammensetzung enthalten ist.

5. Emulsion nach einem der vorstehenden Ansprüche, wobei das Öl aus der Gruppe Dimethicon, Dimethicon und Trimethylsiloxysilicat, Phenyldimethicon, Stearoxydimethicon, Cyclomethicon, Cyclomethicon und Dimethicon oder Kombinationen davon ausgewählt ist.

6. Verfahren zur Herstellung einer konzentrierten Öl-in-Wasser-Emulsion mit den Merkmalen nach einem der Ansprüche 1 bis 5, umfassend:
a) Anlegen hoher Scherkräfte an eine Öl-in-Wasser-Emulsion bildende Materialien, wobei es sich bei 3,0 bis 40 % der Materialien um Öl, das vollständig auf Siliconbasis beruht, und bei 0,02 bis 2,0 Gew.-% der Materialien um ein amphipatisches Emulgiermittel handelt; und
b) Gewinnen einer konzentrierten Öl-in-Wasser-Emulsion mit den Eigenschaften (a) und (b) gemäß der Definition in Anspruch 1.

7. Verfahren zur Herstellung einer verdünnten Öl-in-Wasser-Emulsion, umfassend:
a) Verdünnen einer Zusammensetzung, die einem der Ansprüche 1 bis 5 entspricht oder nach dem Verfahren von Anspruch 6 hergestellt worden ist, mit Wasser unter geringen Scherkräften bis das Wasser in einem Anteil von 70 bis 99 Gew.-% der Zusammensetzung vorliegt; und
b) Gewinnen einer verdünnten Öl-in-Wasser-Emulsion, die stabil ist und eine Viskosität von weniger als 100 Cp (1,0 g/cm x sec) aufweist.

8. Verwendung einer Emulsionszusammensetzung, die einem der Ansprüche 1 bis 5 entspricht oder nach dem Verfahren von Anspruch 6 oder 7 hergestellt worden ist, bei der Herstellung einer Lotion, Creme, Sprühmittels, Gels oder vorbefeuchteten Tuchs ("Baby-Wischtuch") zur Prophylaxe oder Therapie von Windel-Dermatitis.

## Revendications

1. Composition d'émulsion concentrée huile dans eau, dans laquelle l'huile est présente dans le domaine allant de 3,0% à 40% en poids de ladite composition et est entièrement à base de silicone, dans laquelle un agent émulsifiant amphipathique est présent dans le domaine allant de 0,02% à 2,0% en poids de la composition, et dans laquelle l'émulsion a été formée sous des forces de cisaillement élevées, l'émulsion ayant les propriétés combinées selon lesquelles :
(a) elle est stable quand elle est sous forme concentrée; et
(b) diluée dans l'eau sous des forces de cisaillement faibles, elle peut former une émulsion diluée stable huile dans eau dans laquelle l'eau est présente en une quantité de 70 à 99% en poids de ladite composition et qui possède une viscosité inférieure à 100 cps (1,0 g/cm.sec).

2. Emulsion selon la revendication 1, dans laquelle l'agent émulsifiant est choisi dans le groupe constitué par des polymères carboxy vinyle, des copolymères octadécène - anhydride maléique, des copolymères éther stéarylique - anhydride maléique, des copolymères stéarate d'alkyle - anhydride valérique et des copolymères d'acide acrylique substitué par un groupe alkyle en C₁₂-C₂₂ où le groupe alkyle est le groupe laurique, myristique, palmitique, stéarique, oléique, linolénique ou isostéarique, ou des combinaisons.

3. Emulsion selon la revendication 1 ou 2, dans laquelle l'agent émulsifiant est présent dans le domaine allant de 0,8% à 1,4% en poids de la composition.

4. Emulsion selon la revendication 1, 2 ou 3, dans laquelle l'huile est présente dans le domaine allant de 27% à 33% en poids de la composition.

5. Emulsion selon l'une quelconque des revendications précédentes, dans laquelle l'huile est choisie dans le groupe constitué par la diméthicone, la diméthicone et le triméthylsiloxy silicate, la phényl diméthicone, la stéaroxy diméthicone, la cyclométhicone, la cyclométhicone et la diméthicone, ou leurs combinaison.

6. Procédé de préparation d'une émulsion concentrée huile dans eau ayant les caractéristiques de l'une quelconque des revendications 1 à 5, consistant à :
(a) appliquer des forces de cisaillement élevées à des matériaux formateurs d'émulsion huile dans eau, dont 3,0 à 40% des matériaux sont de l'huile, toutes ces huiles étant à base de silicone, et dont 0,02% à 2,0% en poids des matériaux sont un agent émulsifiant amphipathique; et
(b) récupérer une émulsion concentrée huile dans eau ayant les propriétés (a) et (b) définies dans la revendication 1.

7. Procédé de préparation d'une émulsion diluée huile dans eau, consistant à :
(a) diluer à l'eau sous des forces de cisaillement faibles une composition selon l'une quelconque des revendications 1 à 5, ou préparée par le procédé selon la revendication 6, jusqu'à ce que l'eau soit présente en une quantité de 70 à 99% en poids de la composition; et
(b) récupérer une émulsion diluée huile dans eau qui est stable et qui a une viscosité de moins de 100 cps (1,0 g/cm.sec).

8. Utilisation d'une composition d'émulsion comme revendiqué dans l'une quelconque des revendications 1 à 5, ou préparée par le procédé de la revendication 6 ou 7, pour la fabrication d'une lotion, d'une crème, d'un spray, d'un gel ou d'une serviette pré-humidifiée ("couche de bébé") pour la prophylaxie ou le traitement de la dermatite des couches.
